# EUROPEAN PATENT SPECIFICATION

(11) **EP 3 937 829 B1**
(45) Date of publication and mention of the grant of the patent: **03.01.2024**
(21) Application number: 20713754.8
(22) Date of filing: 19.02.2020
(51) Int. Cl.: A61C 7/10

(54) **ORTHODONTIC OR ORTHOPEDIC SCREW FOR DENTISTRY AND ORTHODONTIC OR ORTHOPEDIC APPLIANCE FOR DENTISTRY**
ORTHODONTISCHE ODER ORTHOPÄDISCHE SCHRAUBE FÜR DIE ZAHNHEILKUNDE UND ORTHODONTISCHE ODER ORTHOPÄDISCHE VORRICHTUNG FÜR DIE ZAHNHEILKUNDE
VIS ORTHODONTIQUE OU ORTHOPÉDIQUE POUR DENTISTERIE ET APPAREIL ORTHODONTIQUE OU ORTHOPÉDIQUE POUR DENTISTERIE

(30) Priority: 14.03.2019 IT 201900003741
(43) Date of publication of application: 19.01.2022
(73) Proprietor: Leone S.p.A., 50019 Sesto Fiorentino (Firenze) (IT)
(72) Inventor: SCOMMEGNA, Gabriele, 50029 Tavarnuzze Impruneta (FI) (IT); DOLFI, Maurizio, 50143 Firenze (IT)
(74) Representative: Mincone, Antimo
(86) International application number: PCT/IT2020/050034
(87) International publication number: WO 2020/183506

(56) References cited:
- WO-A1-2016/046798
- DE-A1-102017 102 236
- US-A- 4 571 177
- US-A1- 2010 112 507
- US-A1- 2015 024 334
- US-A1- 2015 056 566

## Description

The present invention relates to an orthodontic screw and a set of elements for making an orthodontic appliance or an orthopedic appliance for dentistry comprising said screw and a process for manufacturing an orthodontic or orthopedic appliance for dentistry. The orthodontic or orthopedic appliance can be of the type made by CAD/CAM techniques.

In orthodontics and dentistry, the use of CAD/CAM methods for the fabrication of orthodontic or orthopedic appliances has become increasingly widespread. According to these methods, there is a joint and integrated use of software systems for computer-aided design (Computer-Aided Design, CAD) and computer-aided manufacturing (Computer-Aided Manufacturing, CAM). The use of integrated CAD/CAM systems makes it easier to transfer information from the first to the second step of the process and has modified the entire production process.

In the case of the manufacturing of an orthodontic or orthopedic appliance, the design of the same appliance is preceded by a three-dimensional scan of the patient's intra-oral cavity by computerized optical detection means. The scan thus performed generates files (generally in the .stl format) which are used for the virtual processing of the orthodontic set-up, i.e. to design by computer the orthodontic appliance to be manufactured using a computer-aided manufacturing process for many of the parts forming the appliance. In other words, virtual modeling is carried out without the traditional realization, by means of dental impression, of a physical model according to which the orthodontic appliance is shaped, with a process in which the manufacture of many parts of the appliance is carried out by means of CAM systems.

In the case of a palatal expander, the portions of the appliance that connect the orthodontic screw to the dental arch or to the palatal bone are formed by CAM, for example by "laser sintering" or "laser melting" procedures, i.e. by additive manufacturing from metal powders. At the end of this process, the connecting portions to the dental arch (i.e. those parts of the appliance which correspond to the orthodontic bands - and which may actually be orthodontic bands or other elements of different shape and size suitable for attachment to the dental arch), as well as the connecting arms to the orthodontic screw, must be connected to the screw by processes which are not standardized because there are no orthodontic screws designed for this use. For this reason, one approach followed by dental technicians is to modify an existing orthodontic screw provided with arms for connecting the screw to the portions of the appliance formed by CAM. In practice, the process of forming an orthodontic appliance involves a mainly computerized phase and a final phase that requires manual intervention by the operator, an intervention that is largely dependent on the skill and experience of the operator himself.

US4571177 discloses an orthodontic screw comprising two bodies connected by positioning means comprising a central screw and two guides parallel to the central screw to allow the distance between said bodies to be adjusted with a reciprocal displacement along a predetermined direction, wherein said bodies have a seat suitable for receiving a portion of a connecting structure associable with the orthodontic screw for its connection to the teeth of a dental arch, and wherein said seat consists of a hole in which parts of the connecting structure are inserted with an insertion movement in an axial direction with respect to the hole. Furthermore, said hole is arranged on the same axis as the guides. Therefore, the orthodontic screw disclosed in US4571177 is disadvantageous both in terms of mechanical stability, because the reactions exerted along the portions of the connecting structure develop along the same axes of the guides, and in terms of assembly complexity and stability of the fastening of the connecting structure to the bodies of the orthodontic screw, because these portions of the connecting structure must be inserted axially in the receiving holes whose dimensions are extremely reduced and the connection is formed only by welds along the outer edge of the hole.

The main purpose of the present invention is to provide an orthodontic screw and a set of elements form manufacturing an orthodontic appliance, i.e. an orthodontic appliance comprising the screw itself, as well as a process for manufacturing an orthodontic or orthodontic appliance able to eliminate the drawbacks of the prior art.

This result has been achieved, in accordance with the present invention, by providing a device having the features indicated in the independent claims. Other features of the invention are the subject of the dependent claims.

Among the advantages offered by the present invention there are, in particular, the following: the possibility to realize an orthodontic or an orthopedic appliance with CAD CAM methodology and to associate to the orthodontic screw the connection structure to the teeth in an effective, safe and quick way, in particular if compared to the known techniques; it is possible to fix the connection arms to the orthodontic screw in different and suitable ways for the type of orthodontic appliance to be formed; the fixing of the connection arms to the orthodontic screw is made much easier by the presence of corresponding open seats in which the ends or portions of the arms can be inserted with a movement along an orthogonal direction to the arms, i.e. along an orthogonal direction to the open seats; the fixing of the arms to the orthodontic screw is remarkably stable because each arm can be fixed, for example by welding, to the receiving seat on a fixing area that extends for a relatively high value, corresponding also to the entire longitudinal extension of the seat; the arms can be fixed to the orthodontic screw in areas not corresponding (and not coaxial) to those in which the guides of the orthodontic screw are present; the simplicity of construction and, therefore, also the relatively low cost; the reliability; the possibility of using biocompatible materials of the type normally used for the construction of intra oral appliances.

These and further advantages and features of the present invention will be more and better understood by every person skilled in the art thanks to the description that follows and to the annexed drawings, provided by way of example but not to be considered in a limitative sense, in which:
- Fig. 1 is a perspective bottom view of a possible embodiment of an orthodontic appliance that is not within the scope of the claims, positioned on the upper arch of an orthodontic model identified with the reference numeral 100;
- Fig. 2 is a perspective bottom view of the embodiment of the appliance shown in Fig. 1 without the orthodontic model;
- Figs. 3, 4, 5 and 6 show a possible embodiments of an orthodontic screw that is not within the scope of the claims, represented, respectively, in a schematic perspective view (Fig. 3), in a schematic front view (Fig. 4), in a schematic top view (Fig. 5), and in a schematic side view (Fig. 6);
- Figs. 7 and 8 show another possible embodiment of an orthodontic screw that is not within the scope of the claims, represented, respectively, in a schematic perspective view (Fig. 7) and in a schematic side view (Fig. 8);
- Figs. 9 and 10 show a possible embodiment of an orthodontic screw according to the invention, represented, respectively, in a schematic perspective view (Fig. 9) and in a schematic side view (Fig. 10);
- Figs. 11 and 12 show yet another possible embodiment of an orthodontic that is not within the scope of the claims, represented, respectively, in a schematic perspective view (Fig. 11) and in a schematic front view (Fig. 12);
- Fig. 13 shows a possible embodiment of a connection structure between the orthodontic screw and the orthodontic bands for the realization of an orthodontic appliance according to the invention, represented in a schematic bottom view;
- Figs. 14, 15, 16 and 17 show another embodiment of a connection structure associable with an orthodontic screw different from that of the present invention, represented in different scales, respectively, in a schematic top view associated with an orthodontic screw (Fig. 14), in a schematic bottom view associated with an orthodontic screw (Fig. 15), in a schematic bottom view without the orthodontic screw (Fig. 16), and in section according to the line XVII-XVII of Fig. 16 with an enlarged detail (Fig. 17);
- Fig. 18 shows the connecting structure of Fig. 13, represented in a perspective bottom view associated with an orthodontic screw different from that of the present invention;
- Figs. 19 and 20 show a further embodiment of the connection structure with additional reinforcement arms, respectively in a perspective view from below (Fig. 19) and in a bottom view (Fig. 20);
- Fig. 21 is a schematic bottom view of a possible embodiment of an orthopedic appliance (F) for dentistry, positioned on the upper arch of an orthodontic model identified with the reference numeral (100);
- Figs. 22 and 23 show a bottom view and a side view of the appliance of Fig.21;
- Fig. 24 is a schematic perspective view from below of a further embodiment of an orthodontic appliance that is not within the scope of the claims, provided with only one pair of connecting arms and positioned on the upper jaw of an orthodontic model identified with the reference numeral (100);
- Figs. 25, 26 and 27 are schematic views of the orthodontic screw used in connection with the example of Fig.24 and represent, respectively, a perspective view (Fig.25), a bottom view (Fig.26) and a side view (Fig.27) of said orthodontic screw;
- Figs. 28 and 29 show a connection structure for connecting the orthodontic screw and the bushings intended to be fixed to the dental arches of Fig.24, represented as a schematic bottom view (Fig.28) and top view (Fig.29);

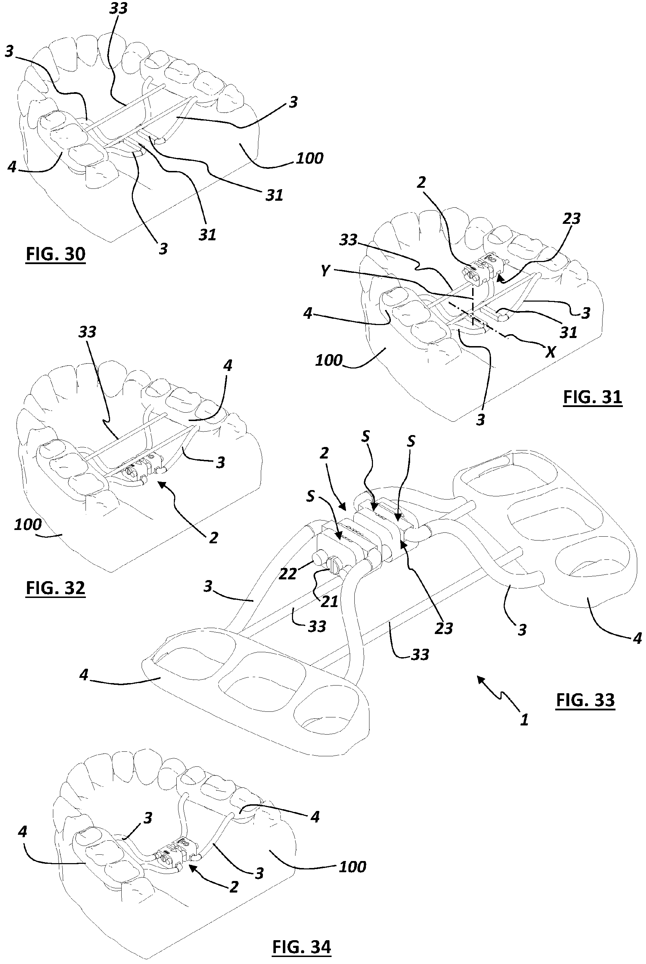

- Figs. 30, 31, 32, 33, 34 are, in a succession of schematic views not all in the same scale, a possible example of association of the orthodontic screw to the arms for forming an orthodontic appliance; in particular, in the Figs. 30-34 it is possible to observe in sequence: the positioning of the structure (3, 4, 33), including the support arms (33), the connecting arms (3) and the orthodontic bands (4), on the model (100) (Fig.30); the positioning of the orthodontic screw (2) on the above mentioned structure (Fig.31); the orthodontic screw (2) positioned on the structure (3, 4, 33) (Fig.32); the orthodontic screw (2) definitively connected to the structure (3, 4, 33) (Fig.33), without the model (110); the group (2, 3, 4), including the orthodontic screw (2), the connecting arms (3) and the orthodontic bands (4), once the support arms (33) have been removed (Fig.34);
- Figs. 35, 36 are schematic representations of possible examples of connection of the arms (3) to the orthodontic screw (2) in which it is shown that the introduction of the arms or their portions or their ends takes place according to a direction (Y) substantially orthogonal to the direction (X) of the same arms (3) in the seats (23) intended for receiving the arms.

Reduced to its essential structure and with reference to the annexed figures, an orthodontic screw (2) in accordance with the present invention is of the type comprising two bodies (20) connected by means of positioning means (21, 22) shaped and arranged in a way to allow adjustment of the distance between said bodies (20) with a movement along a first direction (F).

The screw (2) is used to form an orthodontic appliance (1) through the connection to fixing means to the teeth (4) through one or more connecting arms (3) arranged between the screw (2) and the fixing means to the teeth (4).

The fixing means to the teeth (4), as in the examples of the attached drawings, can consist of a plurality of bushes or orthodontic bands (4) associated with each other and made by means of the CAD/CAM technique. In practice, using the CAD/CAM method, a structure is created that is able to copy the patient's dental arch to be permanently fixed to the same arch and that is provided with a series of arms that allow it to be connected to the orthodontic screw that provides the necessary thrust for the orthodontic treatment. It is understood that, in the context of the present invention, the use of the CAD/CAM technique to create said structure is not strictly necessary since it is still possible to apply the traditional techniques adopted in this sector.

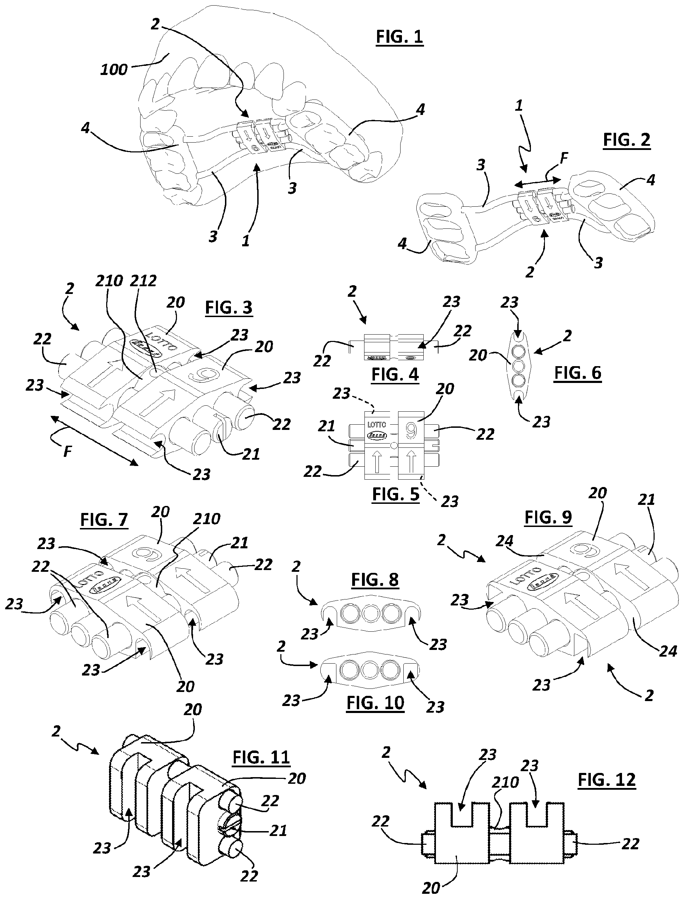

In the present description, the term orthodontic screw identifies a device comprising two bodies connected by positioning means shaped and arranged to allow the adjustment of the distance between said bodies to determine a corresponding thrust during an orthodontic treatment. The positioning means shown in the drawings by way of example are the screw (21) provided with the operating portion (210) and the rectilinear guides (22) parallel to the screw (21). These means can be differently shaped according to the structure and function of the orthodontic screw; for example they may comprise other types of positioning means suitable for providing thrust such as, for example, a screw differently shaped or provided with preloaded elastic means. The term orthodontic appliance means a set of elements designed to influence the shape and/or the function of the stomatognathic system and comprising said screw and a connection or connecting structure to one or more portions of a patient's dental arch. The design and formation of the connection structure (i.e. of the assembly that includes the arms and bands or other means of fixed or temporary bonding to the teeth) between the patient's dental arch and the screw (or other thrust device) is carried out using CAD/CAM techniques. The signs or marks on the bodies (20) of the screw (2), such as the signs or marks visible for example in Fig. 3, Fig. 7 and Fig. 9, can provide indications relating to the production lot, the manufacturer or the commercial name of the product but they are not strictly necessary in the context of the present invention.

The screw (2) is characterized in that it is provided with a seat (23) shaped to accommodate a portion (30; 31) of the arms (3).

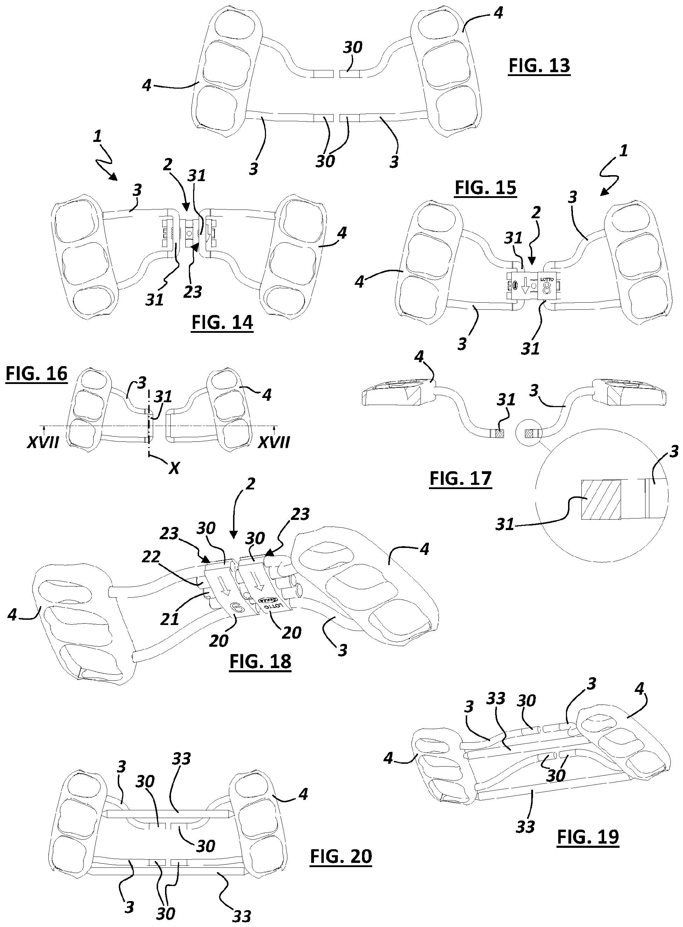

In particular, with reference to Figs. 1-8 and 14-18, the seat (23) is through to allow the acceptance of a portion (31) or of one end (30) of the arms (3). In practice, the arms (3) can have a substantially continuous development, as in the example shown in Figs. 14-17, and an entire portion of the arm is stably received in the seat (23) which extends linearly complementary to the portion (31) of the arm (3).

The seat (23), as shown by way of example in Figs. 9 and 10, is closed at one of its ends to allow the reception of one end (30) of the arms (3). In Fig. 9 are marked with (24) the walls of the body (20) which delimit the closure of the seat (23) at one of its ends.

The seat (23) is can be differently shaped in section; for example, it can have a circular or polygonal cross section; in the case of a polygonal section, the constraint to the relative rotation between the seat (23) and arm (3) is more effective, which will consequently be similarly shaped. For example, as shown in Figs. 9-10, 11-12 and 14-17, the seat (23) has a quadrilateral cross section, like the corresponding portions of the arms (3).

In all the illustrated examples, with the exception of the example shown in Figs. 11-12 and 14-17, the seat (23) develops parallel to the direction (F) of displacement of the bodies (20). On the contrary, in Figs. 11-12 and 14-17, the seat (23) develops orthogonally with respect to said first direction (F).

In the example illustrated in Figs. 3-6, the seat (23) is open laterally being made as a groove that extends along both sides of the bodies (20) placed externally with respect to the guides (22). This type of positioning of the seat (23) is particularly advantageous for associating the arms (3) on the external sides of the screw (2).

In the example illustrated in Figs. 7-8, 9-10 and 11-12, the seat (23) is open at the bottom being made as a groove that extends on the lower face of the bodies (20), or on the face intended to be facing the palate when the appliance is positioned on the upper dental arch. This type of positioning of the seat (23) is particularly advantageous for keeping the portions of the arms (3) connected to the same screw (2) covered by the screw cover (2).

The orthodontic screw (2) can be used to make an orthodontic appliance (1) comprising an orthodontic screw (2) and a connection structure to a dental arch, which includes fixing means to the teeth (4), and one or more connection (3) arranged between said screw (2) and said fixing means to the teeth (4). For example, as shown in the annexed drawings, the means (4) for fixing to the teeth consist of orthodontic bands.

Advantageously, the seat (23) is formed in a direction that does not coincide with the axes of the screw (21) and of the guides (22).

An orthodontic appliance (1) in accordance with the invention is characterized in that it comprises an orthodontic screw (2) made according to the foregoing description.

In particular, the connecting arms (3) are provided with a free end (30) shaped so as to be insertable in the seat (23) in particular when the latter is closed at one of its ends (24).

The connecting arms (3) can be provided with a connecting section (31) shaped to be insertable in a corresponding seat (23). In the examples shown in the figure, said connecting section (31) is substantially orthogonal to the arms (3) connected by it.

In the example shown in Figs. 19 and 20, the structure of the orthodontic appliance is provided with additional reinforcement arms (33) with portions (34) of reduced section that can be easily separated. In this way, the assembly consisting of the bands (4) and the arms (3) is kept together in an arrangement suitable for connection to the screw (2) even if the arms (3) and the bands (4) are separated from each other.

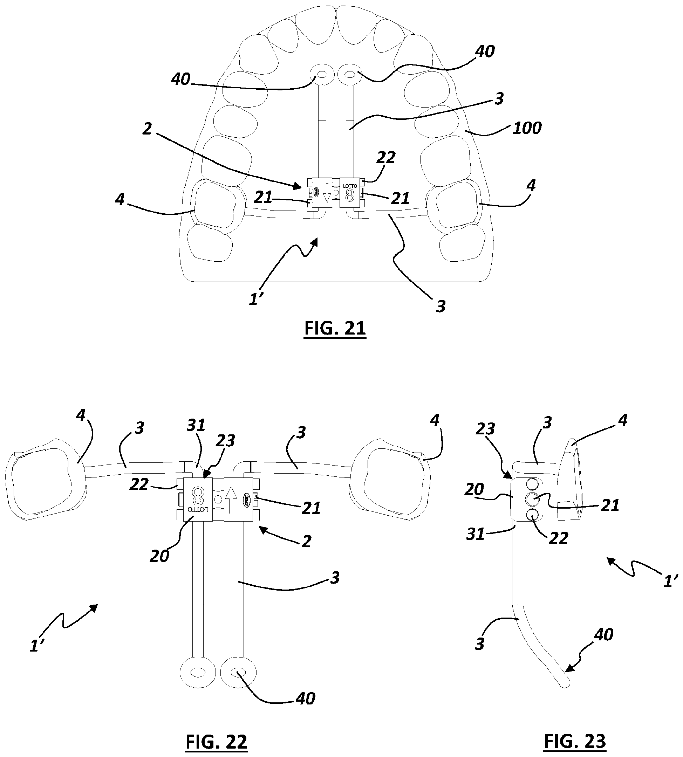

In the example shown in Figs. 21-23, the device (1') is an orthopedic device for dentistry. The apparatus (1') comprising an orthodontic screw (2) and a connection structure to a dental arch comprising means for fixing to the teeth (4) as well as means for fixing (40) to a bone structure. In practice, in a known manner, the apparatus (1'), in addition to the teeth, can be fixed to a bone structure of the patient, for example to the palate, by means of suitable fixing means which, with reference to the example illustrated, can comprise screws which pass through the holes (40) presented by the arms (3) and inserted in corresponding seats provided on the patient's palate. Advantageously, the appliance (1') comprises an orthodontic screw (2) made according to one of the examples previously described. Still with reference to the examples of Figs. 21-23, the portions (31) of the arms (3) are stably accommodated in the seats (23) of the bodies (20) of the screw (2) to simplify the construction of the appliance (1') thanks to the use of a screw (2) which originally does not have arms but is provided with said seats (23) for accommodating the arms (3) themselves.

In Figs. 21-23, the drawings are purely exemplary and provide a graphic representation that does not necessarily correspond to a specific treatment to be performed. For example, the shape and length of the arms could be different from what is shown in the drawings.

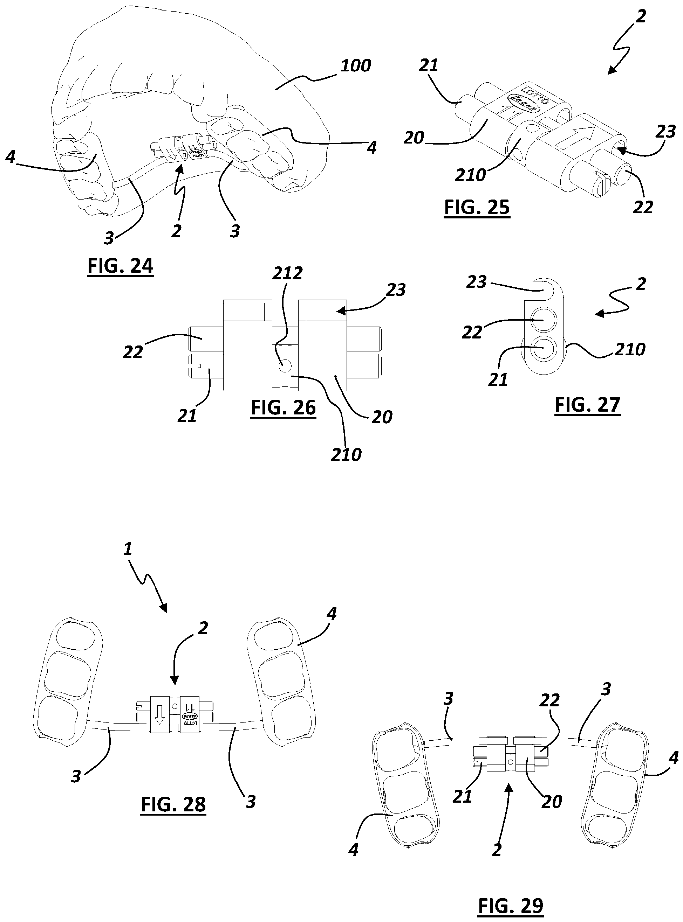

The orthodontic appliance (1) shown in Figs. 24-29 comprises an orthodontic screw (2) and a connection structure to the dental arch comprising fixing means to the teeth (4) and a single connection arm (3) arranged between the screw (2) and each portion for fixing to the teeth (4).

In practice, in this possible embodiment of the invention, only a pair of arms (3) is provided and the screw (2) is corresponding provided with a corresponding pair of seats (23) to accommodate the ends of the two arms (3). The orthodontic screw (2) is of the type comprising an adjustment screw (21), which corresponds to the central screw (21) of the previous examples, and to a single guide (22). Also in this case the conformation of the seats (23) is purely exemplary and also in this case, as in the previous ones, the design and formation of the connection structure can be carried out using CAD / CAM techniques.

From what has been previously expressed and from the attached drawings, it is evident that one of the advantages of the present invention is that the seats (23) are open along their longitudinal development thus allowing the insertion of the arms (3) by means of a movement along a direction (Y) orthogonal or substantially orthogonal to the longitudinal development (X) of the arms themselves, that is, along a direction (Y) orthogonal or substantially orthogonal to the seats (23); in practice, the insertion of the arms (i.e. portions 31 or free ends 30 of the arms themselves) into the seats (23) occurs with a movement which makes the fixing extremely easy and safe for at least two reasons. A first reason relates to the ease with which it is possible to insert an arm in a seat which has a very wide opening by moving the same arm orthogonally with respect to its longitudinal axis; the same ease of insertion is not guaranteed by an axial insertion of the arm in a closed seat, i.e. a seat which has its open insertion part defined only by an axial hole. The second reason relates to the fact that with the seat open longitudinally it is possible to weld the arm (3) to the screw (2) along the entire longitudinal development of the seat (23).

Another advantage from the point of view of the stability of the orthodontic appliance is determined by the fact that the arms (3) can be fixed to the orthodontic screw (2) in areas that do not correspond (and are not coaxial) to those in which the guides (22) of the orthodontic screw are placed.

In the example illustrated in the attached drawings, the screw (21) is provided with a operating portion (210) which has radial holes (212) arranged for the insertion of a special tool thanks to which it is possible to proceed, by rotating the operating portion of an angle that varies according to the desired distance between the bodies (20), with the actuation of the orthodontic screw (2). In other words, said radial holes (212) are configured to be able to house a portion of the tool inside them, the rotation of which involves the actuation of the orthodontic screw and the consequent spacing of the bodies (20), according to the direction indicated with the arrow (F) in the drawings.

In accordance with the example shown in Fig.7-8, said seats (23) are open along the entire lower surface of the bodies (20).

In the example shown in Fig.3 and Fig.6 the seats (23) are open along the entire lateral extension of the bodies (20).

In the present description, the lower term has been used to indicate the side that is at the bottom when the screw 2 is in the position shown in Fig.3 or Fig.9.

These configurations allow, in particular, a better and easier fixing of the connection arms (3) to said seats (23) by welding which prevents their relative movement.

In other words, the open seats (23) arranged for housing the ends (30) of the connecting arms (3) facilitate and make more effective the fixing between said arms (3) and the bodies (20) on which said open seats (23) are formed. The fact that the seats (23) are open for their entire extension considerably increases the ease of fixing the arms (3) to the bodies (20) because the introduction of the arms (3) inside the seats (23) is possible by moving along a radial direction (i.e. orthogonal to the axis of the arms) with an area intended for reception defined by the entire development of the seat (23) capable of being "centered" without any difficulty. On the contrary, if the seats (23) were not open and therefore the introduction of the arms (23) was possible only by means of an axial movement (i.e. in the direction defined by the same axis of the arm 3), the introduction of the free end (30) of the arm (3) in the hole defined by the receiving seat would be extremely difficult or even impossible also because of the possible forces offered by the reaction of the arms to their deformation.

In particular, in the drawings of Figs. 30-34 it is possible to observe in sequence: the positioning of the structure (3, 4, 33), including the support arms (33), the connecting arms (3) and the orthodontic bands (4), on the model (100) (Fig. 30); the positioning of the orthodontic screw (2) on said structure (Fig. 31); the orthodontic screw (2) positioned on the structure (3, 4, 33) (Fig. 32); the orthodontic screw (2) definitively connected to the structure (3,4,33) (Fig. 33); the assembly (2, 3, 4), comprising the orthodontic screw (2), the connecting arms (3) and the orthodontic bands (4), once the support arms (33) have been removed (Fig. 34).

In particular, Fig. 33 shows the entire configuration of the orthodontic screw (2) connected to said structure, and it is possible to observe the welding (S) carried out along the development of the open seats (23) inside which the portions (31) of the connecting arms (3) pass. For the technician, the realization of the weld in such configuration is much more comfortable and guarantees a more extensive fixing of the connection arms (3) to the open seats (23). In other words, the welding between said arms and said open seats is easier to implement as well as more extensive, with undoubted advantages both from the manufacturing point of view and from that of increasing the resistance to mechanical stress.

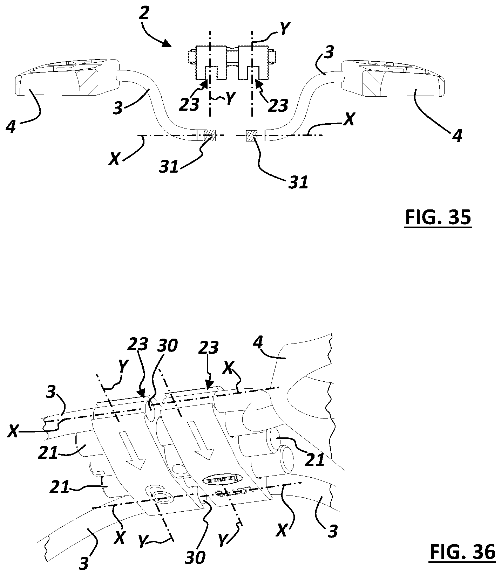

In Figs. 35 and 36 the reference (X) indicates the longitudinal axis of the portions (31) of the arms (3) and of the ends (30) of the arms (3) intended to be introduced into the seats (23) of the orthodontic screw (2). In practice, the reciprocal approach movement between the arms (3) and the seats (23), necessary for fixing, takes place in a direction (Y) which is orthogonal to that of the axes (X) and which allows the introduction into the seats (23) through the open longitudinal portions of the same seats (23).

In relation to what has been described and illustrated, an orthodontic screw in accordance with the present invention comprises two bodies (20) connected by means of positioning means (21 , 22) designed to allow the distance between said bodies (20) to be adjusted with a mutual displacement along a predetermined direction (F), wherein said bodies (20) have a seat (23) adapted to receive a portion (30, 31) of a connection structure (3, 4) associable with the orthodontic screw for its connection to the teeth of a dental arch, wherein said seat (23) consists of a groove (23) formed on an external surface of the bodies (20).

In this way, said portion (30, 31) of said connection structure (3, 4) is inserted in said seat (23) through a displacement (Y) orthogonal to the same seat (23).

In the examples previously described, the structure (3, 4) for connecting the orthodontic screw to the teeth of the dental arch is formed by the arms (3) and the orthodontic bands (4) arranged on the arms themselves, and said portion (30, 31) is consisting of a portion of the arms (3). In the examples previously described, the arms (3) are elements oriented parallel to the seats (23) whereby said displacement (Y) is an orthogonal or substantially orthogonal displacement to the seats (23).

In conclusion, it is possible to affirm that the invention therefore relates to an orthodontic screw (2) comprising two bodies (20) connected by means of positioning means (21, 22) adapted to allow the distance between said bodies (20) with a reciprocal movement along a predetermined direction (F), in which
said bodies (20) have a seat (23) suitable for receiving a portion (30, 31) of a connecting structure (3, 4) associated with the orthodontic screw for its connection to the teeth of a dental arch; the screw (2) is characterized in that said seat (23) is provided by a groove (23) formed on an external surface of the bodies (20), so that said portion (30, 31) of said connection structure (3 , 4) can be inserted in said seat (23) through a displacement (Y) orthogonal to the same seat (23).

The seat (23) is closed at one of its ends (24), and can be of polygonal cross section, in particular also quadrilateral.

The seat (23) can be develop parallel or orthogonally with respect to said direction (F) and is formed along a direction not coincident with the axis of the screw (21) and guides (22).

The invention also relates to an in orthodontic appliance (1) comprising an orthodontic screw (2) and a connecting structure (3, 4) to the teeth of a dental arch comprising means (4) for fixing to the teeth and one or several connecting arms (3) arranged between said orthodontic screw (2) and said fixing means to the teeth (4); the appliance (1) is characterized in that it comprises an orthodontic screw (2) made as described above. Furthermore, each of said connecting arms (3) can be provided with a portion or section (31) shaped to be insertable in said seat (23) or with a free end (30) shaped to be insertable in said seat (23).

One or more additional connecting elements (33) can be provided to keep said fixing means (4) to the teeth and said arms (3) together.

The fixing means (4) to the teeth as well as the connecting arms (3) can be carried out by means of CAD-CAM technique.

In the same way, it is an object of the present invention, an orthopedic appliance for dentistry (F) comprising an orthodontic screw (2) and a structure (3, 4) for connecting to a dental arch comprising means (4) for fixing to the teeth of a dental arch and fixing means (40) to a bone structure and one or more connecting arms (3) arranged between said orthodontic screw (2) and said fixing means to the teeth (4) and said fixing means (40) to a bone structure; also in this case the appliance ( 1' ) is characterized in that it comprises an orthodontic screw (2) made as described above. In addition, the fastening means to the teeth (4) and said connecting arms (3) can be carried out by means of CAD-CAM technique.

The invention also relates to a process for the construction of an orthodontic appliance (1) or an orthopedic appliance (G) for dentistry comprising:
- an orthodontic screw (2) provided with two bodies (20) connected by positioning means (21, 22) shaped and arranged to allow the adjustment of the distance between said bodies (20) with a movement along a first direction (F); and
- a connection structure (3, 4) that can be associated with the orthodontic screw (2) for its connection to the teeth of a dental arch.

The method is characterized in forming on an external surface of the bodies (20) of said screw a seat (23) consisting of a groove (23), and introducing a portion (30, 31) of said connection structure (3, 4) in said seat (23) through a displacement (Y) orthogonal to the same seat (23). Furthermore, the connection structure (3, 4) can be carried out by using the CAD-CAM technique.

## Claims

1. Orthodontic screw comprising two bodies (20) connected by means of positioning means (21, 22) adapted to allow the distance between said bodies (20) to be adjusted with a reciprocal movement along a predetermined direction (F), wherein said bodies (20) have a seat (23) adapted to receive a portion (30, 31) of a connecting structure (3, 4) associable with the orthodontic screw for its connection to the teeth of a dental arch, wherein said seat (23) consists of a groove (23) formed on an external surface of the bodies (20), so that said portion (30, 31) of said connecting structure (3, 4) can be inserted in said seat (23) through a displacement (Y) orthogonal to the same seat (23), **characterized in that** said seat (23) is closed at one of its ends (24) and **in that** said seat (23) develops parallel to said direction (F).

2. Orthodontic screw according to claim 1, **characterized in that** said seat (23) has a polygonal cross section.

3. Orthodontic screw according to one of the previous claims, **characterized in that** said seat (23) has a quadrilateral cross section.

4. Orthodontic screw according to one of the previous claims, wherein said positioning means consist of a screw (21) and one or more rectilinear guides (22) parallel to the screw (21), **characterized in that** said seat (23) is formed in a direction that does not coincide with the axes of the screw (21) and of the guides (22).

5. Orthodontic appliance (1) comprising the orthodontic screw (2) according to any of the preceding claims and a connecting structure (3, 4) for connection to the teeth of a
dental arch comprising means (4) for fixing to the teeth and one or more connecting arms (3) arranged between said orthodontic screw (2) and said fixing means to the teeth (4).

6. Orthopedic appliance for dentistry (1') comprising an orthodontic screw (2) according to any of claims from 1 to 4 and a connecting structure (3, 4) to the teeth of a dental arch comprising means (4) for fixing to the teeth of a dental arch, fixing means (40) to a bone structure and one or more connecting arms (3) arranged between said orthodontic screw (2), said fixing means to the teeth (4) and said fixing means (40) to a bone structure.

7. Orthodontic appliance (1) comprising an orthodontic screw (2) according to claim 1 and a connecting structure to a dental arch comprising fixing means to the teeth (4) and one or more connecting arms (3) arranged between said screw (2) and said fixing means (4) to the teeth, appliance (1) **characterized in that** each of said connection arms (3) is provided with a portion or section (31) shaped to be insertable in said seat (23).

8. Orthodontic appliance (1) comprising an orthodontic screw (2) according to claim 1 and a connecting structure to a dental arch comprising fastening means to the teeth (4) and one or more connecting arms (3) arranged between said screw (2) and said fixing means (4) to the teeth, appliance (1) **characterized in that** each of said connection arms (3) are provided with a free end (30) shaped to be insertable in said seat (23).

9. Orthodontic appliance (1) according to one of claims 5, 7 or 8, **characterized in that** it is provided with one or more additional connection elements (33) able to keep together said fixing means (4) and said arms (3).

10. Orthodontic appliance (1) comprising the orthodontic screw according to one of claims 1 to 4 and a connecting structure to a dental arch comprising fixing means to the teeth (4) and one or more connecting arms (3) arranged between said screw (2) and said fixing means to the teeth (4), **characterized in that** said fixing means to the teeth (4) and said one or more connecting arms (3) are realized by CAD-CAM technique.

11. Orthopedic appliance (1') for dentistry comprising the orthodontic screw according to one of claims from 1 to 4 and a connecting structure to a dental arch comprising fixing means to the teeth (4) and one or more connection arms (3) arranged between said screw (2) and said fixing means to the teeth (4), **characterized in that** said fastening means to the teeth (4) and said one or more connecting arms (3) are realized by CAD/ CAM technique.

12. Process for making an orthodontic appliance (1) or an orthopedic appliance for dentistry (1') comprising:
- making an orthodontic screw (2);
- making a connecting structure (3, 4) associated with the orthodontic screw (2) for its connection to the teeth of a dental arch;
the process being **characterized in that** said orthodontic screw (2) is the screw according to claim 1 by forming a seat (23) consisting of a groove (23), on a external surface of the bodies (20) of said screw, and by introducing a portion (30, 31) of said connecting structure (3, 4) in said seat (23) through a displacement (Y) orthogonal to the same seat (23).

13. Process for the realization of an orthodontic appliance (1) or an orthopedic appliance (1') for dentistry according to claim 12, **characterized in that** said connecting structure (3, 4) is realized by the CAD-CAM technique.

## Patentansprüche

1. Orthodontische Schraube, umfassend zwei Körper (20), die mittels Positionierungsmitteln (21, 22) verbunden sind, die dazu angepasst sind, zu ermöglichen, dass der Abstand zwischen den Körpern (20) durch eine Hin- und Herbewegung entlang einer vorbestimmten Richtung (F) eingestellt wird, wobei die Körper (20) einen Sitz (23) aufweisen, der dazu angepasst ist, einen Teil (30, 31) einer Verbindungsstruktur (3, 4) aufzunehmen, die mit der orthodontischen Schraube für deren Verbindung mit den Zähnen eines Zahnbogens verbindbar ist, wobei der Sitz (23) aus einer Nut (23) besteht, die auf einer Außenfläche der Körper (20) ausgebildet ist, so dass der Teil (30, 31) der Verbindungsstruktur (3, 4) in den Sitz (23) durch eine Verschiebung (Y) orthogonal zum gleichen Sitz (23) eingeführt werden kann, **dadurch gekennzeichnet, dass** der Sitz (23) an einem seiner Enden (24) geschlossen ist und dass sich der Sitz (23) parallel zur Richtung (F) entwickelt.

2. Orthodontische Schraube nach Anspruch 1,
**dadurch gekennzeichnet, dass** der Sitz (23) einen vieleckigen Querschnitt aufweist.

3. Orthodontische Schraube nach einem der vorhergehenden Ansprüche, **dadurch gekennzeichnet, dass** der Sitz (23) einen viereckigen Querschnitt aufweist.

4. Orthodontische Schraube nach einem der vorhergehenden Ansprüche, wobei die Positionierungsmittel aus einer Schraube (21) und einer oder mehreren geradlinigen Führungen (22) parallel zur Schraube (21) bestehen, **dadurch gekennzeichnet, dass** der Sitz (23) in einer Richtung ausgebildet ist, die nicht mit den Achsen der Schraube (21) und der Führungen (22) übereinstimmt.

5. Orthodontische Vorrichtung (1), umfassend die orthodontische Schraube (2) nach einem der vorhergehenden Ansprüche und eine Verbindungsstruktur (3, 4) zur Verbindung mit den Zähnen eines Zahnbogens, die Mittel (4) zur Befestigung an den Zähnen und ein oder mehrere Verbindungsarme (3) umfasst, die zwischen der orthodontischen Schraube (2) und den Befestigungsmitteln an den Zähnen (4) angeordnet sind.

6. Orthopädische Vorrichtung für die Zahnheilkunde (1`), umfassend eine orthodontische Schraube (2) nach einem der Ansprüche 1 bis 4 und eine Verbindungsstruktur (3, 4) zu den Zähnen eines Zahnbogens, die Mittel (4) zur Befestigung an den Zähnen eines Zahnbogens, Befestigungsmittel (40) an einer Knochenstruktur und einen oder mehrere Verbindungsarme (3) umfasst, die zwischen der orthodontischen Schraube (2), den Befestigungsmitteln an den Zähnen (4) und den Befestigungsmitteln (40) an einer Knochenstruktur angeordnet sind.

7. Orthodontische Vorrichtung (1), umfassend eine orthodontische Schraube (2) nach Anspruch 1 und eine Verbindungsstruktur zu einem Zahnbogen, die Befestigungsmittel an den Zähnen (4) und einen oder mehrere Verbindungsarme (3) umfasst, die zwischen der Schraube (2) und den Befestigungsmitteln (4) an den Zähnen angeordnet sind, wobei die Vorrichtung (1) **dadurch gekennzeichnet ist, dass** jeder der Verbindungsarme (3) mit einem Teil oder Abschnitt (31) versehen ist, der so geformt ist, dass er in den Sitz (23) eingeführt werden kann.

8. Orthodontische Vorrichtung (1), umfassend eine orthodontische Schraube (2) nach Anspruch 1 und eine Verbindungsstruktur zu einem Zahnbogen, die Befestigungsmittel an den Zähnen (4) und einen oder mehrere Verbindungsarme (3) umfasst, die zwischen der Schraube (2) und den Befestigungsmitteln (4) an den Zähnen angeordnet sind, wobei die Vorrichtung (1) **dadurch gekennzeichnet ist, dass** jeder der Verbindungsarme (3) mit einem freien Ende (30) versehen ist, das so geformt ist, dass es in den Sitz (23) eingeführt werden kann.

9. Orthodontische Vorrichtung (1) nach einem der Ansprüche 5, 7 oder 8, **dadurch gekennzeichnet, dass** sie mit einem oder mehreren zusätzlichen Verbindungselementen (33) versehen ist, die in der Lage sind, die Befestigungsmittel (4) und die Arme (3) zusammenzuhalten.

10. Orthodontische Vorrichtung (1), umfassend die orthodontische Schraube nach einem der Ansprüche 1 bis 4 und eine Verbindungsstruktur zu einem Zahnbogen, die Befestigungsmittel an den Zähnen (4) und einen oder mehrere Verbindungsarme (3) umfasst, die zwischen der Schraube (2) und den Befestigungsmitteln an den Zähnen (4) angeordnet sind, **dadurch gekennzeichnet, dass** die Befestigungsmittel an den Zähnen (4) und der eine oder die mehreren Verbindungsarme (3) durch CAD-CAM-Technik realisiert sind.

11. Orthopädische Vorrichtung (1`) für die Zahnheilkunde, umfassend eine orthodontische Schraube nach einem der Ansprüche 1 bis 4 und eine Verbindungsstruktur zu einem Zahnbogen, die Befestigungsmittel an den Zähnen (4) und einen oder mehrere Verbindungsarme (3) umfasst, die zwischen der Schraube (2) und den Befestigungsmitteln an den Zähnen (4) angeordnet sind, **dadurch gekennzeichnet, dass** die Befestigungsmittel an den Zähnen (4) und der eine oder die mehreren Verbindungsarme (3) durch CAD-CAM-Technik realisiert sind.

12. Verfahren zum Herstellen einer orthodontischen Vorrichtung (1) oder einer orthopädischen Vorrichtung für die Zahnheilkunde (1`), umfassend:
- Herstellen einer orthodontischen Schraube (2);
- Herstellen einer Verbindungsstruktur (3, 4), die mit der orthodontischen Schraube (2) für deren Verbindung mit den Zähnen eines Zahnbogens verbunden ist,
wobei das Verfahren **dadurch gekennzeichnet ist, dass** es sich bei der orthodontischen Schraube (2) um die Schraube nach Anspruch 1 handelt, die durch Ausbilden eines aus einer Nut (23) bestehenden Sitzes (23) auf einer Außenfläche der Körper (20) der Schraube und durch Einführen eines Teils (30, 31) der Verbindungsstruktur (3, 4) in den Sitz (23) durch eine Verschiebung (Y) orthogonal zu demselben Sitz (23) hergestellt wird.

13. Verfahren zur Realisierung einer orthodontischen Vorrichtung (1) oder einer orthopädischen Vorrichtung (1') für die Zahnheilkunde nach Anspruch 12, **dadurch gekennzeichnet, dass** die Verbindungsstruktur (3, 4) durch die CAD-CAM-Technik realisiert wird.

## Revendications

1. Vis orthodontique comprenant deux corps (20) raccordés au moyen de moyens de positionnement (21, 22) conçus pour permettre le réglage de la distance entre lesdits corps (20) avec un mouvement réciproque le long d'une direction prédéfinie (F), lesdits corps (20) comportant un siège (23) conçu pour recevoir une partie (30, 31) d'une structure de raccordement (3, 4) pouvant être associée à la vis orthodontique pour son raccordement aux dents d'une arcade dentaire, ledit siège (23) étant constitué d'une rainure (23) formée sur une surface externe des corps (20), de sorte que ladite partie (30, 31) de ladite structure de raccordement (3, 4) puisse être insérée dans ledit siège (23) par un déplacement (Y) orthogonal à ce même siège (23), **caractérisé en ce que** ledit siège (23) est fermé au niveau de l'une de ses extrémités (24) et **en ce que** ledit siège (23) se développe parallèlement à ladite direction (F).

2. Vis orthodontique selon la revendication 1, **caractérisé en ce que** ledit siège (23) comporte une section transversale polygonale.

3. Vis orthodontique selon l'une des revendications précédentes, **caractérisée en ce que** ledit siège (23) comporte une section transversale quadrilatérale.

4. Vis orthodontique selon l'une des revendications précédentes, lesdits moyens de positionnement étant constitués d'une vis (21) et d'un ou plusieurs guides rectilignes (22) parallèles à la vis (21), **caractérisée en ce que** ledit siège (23) est formé suivant une direction qui ne coïncide pas avec les axes de la vis (21) et des guides (22).

5. Appareil orthodontique (1) comprenant la vis orthodontique (2) selon l'une quelconque des revendications précédentes et une structure de raccordement (3, 4) permettant le raccordement aux dents d'une arcade dentaire comprenant des moyens (4) permettant la fixation aux dents et un ou plusieurs bras de raccordement (3) disposés entre ladite vis orthodontique (2) et lesdits moyens de fixation aux dents (4).

6. Appareil orthopédique dentaire (1') comprenant une vis orthodontique (2) selon l'une quelconque des revendications 1 à 4 et une structure de raccordement (3, 4) aux dents d'une arcade dentaire comprenant des moyens (4) permettant la fixation aux dents d'une arcade dentaire, des moyens de fixation (40) à une structure osseuse et un ou plusieurs bras de raccordement (3) disposés entre ladite vis orthodontique (2), lesdits moyens de fixation aux dents (4) et lesdits moyens de fixation (40) à une structure osseuse.

7. Appareil orthodontique (1) comprenant une vis orthodontique (2) selon la revendication 1 et une structure de raccordement à une arcade dentaire comprenant des moyens de fixation aux dents (4) et un ou plusieurs bras de raccordement (3) disposés entre ladite vis (2) et lesdits moyens de fixation (4) aux dents, l'appareil (1) étant **caractérisé en ce que** chacun desdits bras de raccordement (3) est muni d'une partie ou section (31) formée de manière à pouvoir être insérée dans ledit siège (23).

8. Appareil orthodontique (1) comprenant une vis orthodontique (2) selon la revendication 1 et une structure de raccordement à une arcade dentaire comprenant des moyens d'attache aux dents (4) et un ou plusieurs bras de raccordement (3) disposés entre ladite vis (2) et lesdits moyens de fixation (4) aux dents, l'appareil (1) étant **caractérisé en ce que** chacun desdits bras de raccordement (3) est muni d'une extrémité libre (30) formée de manière à pouvoir être insérée dans ledit siège (23).

9. Appareil orthodontique (1) selon l'une des revendications 5, 7 ou 8, **caractérisé en ce qu'**il est muni d'un ou plusieurs éléments de raccordement supplémentaires (33) pouvant maintenir ensemble lesdits moyens de fixation (4) et lesdits bras (3).

10. Appareil orthodontique (1) comprenant la vis orthodontique selon l'une des revendications 1 à 4 et une structure de raccordement à une arcade dentaire comprenant des moyens de fixation aux dents (4) et un ou plusieurs bras de raccordement (3) disposés entre ladite vis (2) et lesdits moyens de fixation aux dents (4), **caractérisée en ce que** lesdits moyens de fixation aux dents (4) et lesdits un ou plusieurs bras de raccordement (3) sont réalisés par une technique CAD-CAM.

11. Appareil orthopédique (1') pour dentisterie comprenant la vis orthodontique selon l'une des revendications 1 à 4 et une structure de raccordement à une arcade dentaire comprenant des moyens de fixation aux dents (4) et un ou plusieurs bras de raccordement (3) disposés entre ladite vis (2) et lesdits moyens d'attache aux dents (4), **caractérisé en ce que** lesdits moyens de fixation aux dents (4) et lesdits un ou plusieurs bras de raccordement (3) sont réalisés par une technique CAD-CAM.

12. Procédé de fabrication d'un appareil orthodontique (1) ou d'un appareil orthopédique pour dentisterie (1') comprenant :
- la fabrication d'une vis orthodontique (2) ;
- la réalisation d'une structure de raccordement (3, 4) associée à la vis orthodontique (2) pour son raccordement aux dents d'une arcade dentaire ;
le procédé étant **caractérisé en ce que** ladite vis orthodontique (2) est la vis selon la revendication 1 fabriquée en formant un siège (23) constitué d'une rainure (23), sur une surface externe des corps (20) de ladite vis, et en introduisant une partie (30, 31) de ladite structure de raccordement (3, 4) dans ledit siège (23) par un déplacement (Y) orthogonal à ce même siège (23).

13. Procédé de réalisation d'un appareil orthodontique (1) ou d'un appareil orthopédique (1') pour dentisterie selon la revendication 12, **caractérisé en ce que** ladite structure de raccordement (3, 4) est réalisée par la technique CAD-CAM.
